# EUROPEAN PATENT APPLICATION

(11) **EP 2 113 268 A1**
(43) Date of publication of application: **04.11.2009**
(21) Application number: 08155464.4
(22) Date of filing: 30.04.2008
(51) Int. Cl.: A61M 5/142, H03K 17/955, H03K 17/96

(54) **Administering device with safety features**

(71) Applicant: F. Hoffmann-La Roche AG, 4070 Basel (CH); Roche Diagnostics GmbH, 68305 Mannheim (DE)
(72) Inventor: Friedli, Kurt, 3421 Lyssach (CH)
(74) Representative: Schwabe - Sandmair - Marx

(57) **Abstract**

The present invention is directed to an administering device (10) for supplying an injectable or infusible product into an organism, in particular a patient, wherein the device comprises a reservoir for storing the product and a supply device for supplying the product from the reservoir into the organism, wherein the device further comprises a non-contact sensor or touch sensor (20) for generating an activation signal for initiating a function of the device.

## Description

The present invention relates to an administering device for supplying an injectable or infusible product into an organism, in particular a patient, according to the preamble in claim 1. In particular, the invention relates to an infusion device for therapeutic applications, for example a portable infusion device, which can be adapted for self-administering medicines such as insulin.

Administering devices or self-administering devices comparable to the device in accordance with the present invention are used in particular to serve diabetes patients. An example of a typical device which forms part of the background art is referred to in WO 2006/114288, which is incorporated into the present disclosure by reference for the purpose of disclosing in principle the basic constructive portions which the basic purpose of the device.

Usually, an administering device is placed in a patient's pocket and connected to the patient's organism to supply a suitable dose of medicine, in particular insulin, to the patient. The user interface which is provided for the patient usually consists of a display and keys or buttons for entering instructions for a control circuit included in the device. By using the display, it is possible to request pre-set information or to view instructions just entered using the keys and/or buttons.

Since the administering device usually works automatically through the control of a control device, it is not necessary for the display to be constantly active. The display requires a lot of electrical energy, hence if the display is in operation for a long time, it is necessary to include large batteries or accumulators in the device. Accordingly, an attempt should be made to activate a display only when it is needed.

On the other hand, in case the display has to be activated, it is necessary to dedicate the additional function to an existing button, wherein a multiplicity of buttons are necessary and this is an obstacle to minimize the size of the overall device.

A comparable problem occurs in case of prior art administering devices which require keys or buttons for programming or for requesting the display of information on a small LCD screen or the like. Since the administering device is usually taken with the patient in the pockets of clothing, it is possible that the keys or buttons are activated arbitrarily and that supplementary doses are administered without a reason. In this respect, it has to be taken into account that an administering device in accordance with the prior art, as also administering device in accordance with the invention has to administer a number of pre-adjustable doses through the day, which have to be administered in accordance with the therapeutic needs of the patient. On the other hand, the patient may need additional doses depending on particular circumstances and events which will vary from day to day, such that for instance a diabetes patient needs additional doses several times a day, the administering of which may have to be initiated using the keys or buttons which are provided in order to operate the device. In this respect, it is possible for the keys or buttons which are provided for programming the basic doses, the so-called basal doses, and which can also be configured for inputting the additional doses, the so-called bolus doses or correcting bolus, to be activated by mistake, such that a dose of medicine may be administered which has no therapeutic effect, but may have severe consequences for the patent. To prevent unintentional doses from being inadvertently administered in this way, the prior-art administering device is provided with at least two keys or buttons which have to be activated in sequence and within a particular time in order to release a bolus does.

Another problem may occur if a button becomes instable, e.g, has a loose connection or the like since this again may cause unintended administration of a large dose with all negative consequences.

Again, however, arranging a key or button which can be handled easily by the patient requires a lot of space. A corresponding prior art device is disclosed in DE-A-103 25 106.

Accordingly, to have additional benefit of an easy transport and a low weight of the administering device, it is necessary to minimize the size of the device, which is a motivation to omit as many keys, switches or buttons, necessary for controlling the administering device, as possible.

Accordingly, it is an object of the present invention to minimise the size and weight of an administering device, in particular an infusion pump device which is used in particular to infuse insulin.

Furthermore, it is another object of the present invention to enable an administering device which is configured to consume less energy, but which can be operated and controlled more safely.

It is the object of the present invention to improve the prior-art administering device. In particular, the administering device of the present invention should be miniaturised, made safer or should be enabled to consume less energy.

In accordance with the present invention, the benefits of the administering device of the present invention are based on a device which is characterised by a non-contact sensor or touch sensor for generating an activation signal for initiating a function of the device.

By means of such a sensor which generates an activation signal, it is possible to either activate a display or screen of the device, or to enable a button or key so that instructions or information can be entered into the device by a patient.

Accordingly, it is possible to save the space for a safety button provided to allow a bolus dose of a medicine, e.g. insulin, to be initiated. The non-contact sensor or touch sensor is able to sense the approach of an earthed object such as a patient's finger and can therefore enable the button or key of the device to allow the device to be instructed to administer a bolus dose. A further benefit is that the operation of such a device by the user can be handled much simpler.

The intention is of course for other instructions or requests for status information on the device to also only be entered when the button is enabled by the activation signal outputted by the non-contact sensor or touch sensor.

Another benefit of the invention is that the activation signal can also activate the display or screen of the device in accordance with the present invention, such that the energy consumption can be reduced such that the battery or accumulator of the device can be reduced in size or the device can be operated for much longer using the same battery or accumulator.

In accordance with one preferred embodiment of the invention, it is possible to incorporate the non-contact sensor or touch sensor into the button itself, such that it is not necessary to provide an additional area on the housing of the administering device of the present invention, which is made of a soft material portion in order on the one hand to enable it to be waterproof, and also for generating an electrical, magnetic or electromagnetic field which can interact with an earthed object, such as an operator or patient's finger, which is approaching the button in order for example to enter some instructions or requests into the device of the present invention via a button. The non-contact sensor or touch sensor can be a capacitive or inductive sensor means, such as is available from companies such as ANALOG DEVICE, CYPRESS, MAXIM and others. Furthermore, it is possible to use sensors which work on the basis of changes in resistance or in a conduction value. Another option would be to use light-sensitive sensors for outputting the activation signal.

The device according to the invention can also be configured to have a set of multiple buttons and a non-contactor sensor or touch sensor may be incorporated into one or multiple of those buttons. Accordingly, this embodiment of the device may comprise more than one non-contact sensor or touch sensor.

However, the preferable option in accordance with the invention seems to be the capacitive or inductive sensor. This is because it is unlikely that objects other than the earthed finger of an operator could trigger a non-contact sensor or touch sensor for generating an activation signal. Further advantages can be achieved by using a control device which enables and/or disables functions of the device within a predetermined time after receiving the activation signal. For instance, if an activation signal is received and the button is not used within a predetermined period of time, the button is deactivated because it is very likely that the operator or patient activated the non-contact sensor or touch sensor unintentionally. On the other hand, if the button has not been activated for a predetermined period of time, it is very likely that the activated display or screen can for example be deactivated, since the patient no longer seems to be currently interested in the information displayed.

Furthermore, it may be advantageous if the activation and/or deactivation of the non-contact sensor or touch sensor and/or button and/or display is communicated to a user or operator of the device, i.e. the patient. For example, it would be possible to output a sound signal, for instance when the non-contact sensor or touch sensor is activated and outputs the activation signal. Furthermore, it may be possible to outputs another sound signal when the button is activated or in particular when the button is deactivated. The same may be expedient with respect to the display.

According to the invention, a button can be provided with an incorporated non-contact sensor or touch sensor such that a first function of the device (e.g., switching on the backlight) is operated and a second function of the device (e.g. bolus drug administration) can be activated if the button is pressed with the non-contact-sensor or touch sensor.

It is of course also possible to output signals other than sound signals, for instance visible signals, vibration signals, buzzer signals or the like, which the user or operator will notice even when the device in accordance with the invention is placed in a pocket of the patient's clothing. Furthermore, it might be advantageous if the type of activation is communicated to the operator or patient. For instance, the type of signal outputted in the event of activation may be different for an activation signal which is within a predetermined range of values. If the non-contact sensor or touch sensor detects a detectable event which is not within a predetermined range, it is possible for a recognisable signal to be outputted in order to inform the operator or patient that something has happened which was close to activating the device to accept instructions inputted using the button. If the event detected by the noun-contact sensor or touch sensor is suitable to allow a signal which is within the predetermined range, the activation signal is outputted and another type of recognisable signal can be brought to the patient's attention. Accordingly, the patient can learn what sort of events can affect the device in an arbitrary manner and should be avoided. Thus, for example, events such as putting keys into the pocket could be identified as actions which should be avoided because the keys could activate the button which is suitable for administering bolus doses.

It is of course also possible to program the controller such that a particular code has to be inputted via the non-contact sensor or touch sensor first, in order to activate the button or the display or other parts or functions of the device of the invention. For instance, one touch on the non-contact sensor or touch sensor could activate the display and two touches could activate the button itself, if the touches are applied to the sensor in sequence and within a certain time interval or time period. Any type of "touch codes" could of course be used, providing they are not too complicated for the operator or patient.

It is also possible to use a "touch code" to activate the administering device or a function of the administering device and to then enter data or request data, also by touching or otherwise interacting with the non-contact sensor or touch sensor, such that any type of button could be omitted and the size and weight of the device, for example an insulin pump, could be even further reduced.

In the following, particular and advantageous embodiments of the present invention are described in more detail with reference to the accompanying drawings. In the drawings:
- Figure 1: shows a schematic representation of a first embodiment of the present invention;
- Figures 2a and 2b: show a second embodiment in a plan view (Figure 2a) and in a partial sectional view (Figure 2b);
- Figure 3: shows functional parts of a third embodiment;
- Figure 4: shows functional parts of a fourth embodiment;
- Figure 5: shows functional parts of a fifth embodiment; and
- Figure 6: shows functional parts of a sixth embodiment.

In the drawings, the same reference numbers have been assigned to identical parts or parts which have at least substantially the same function. Accordingly, the respective parts need not be explained anew in connection with each figure, since the explanation of a part in one figure can be applied to other figures.

In the following, the administering device in accordance with the different embodiments of the present invention is called an "infusion device" for short.

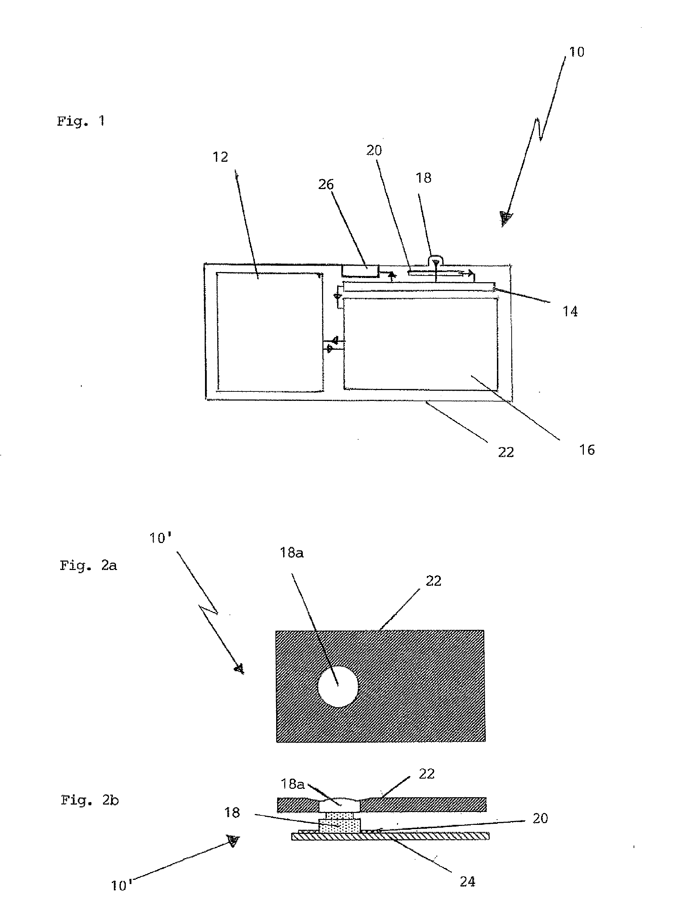

In accordance with Figure 1, an infusion device 10 includes an infusion section 12 which usually consists of a reservoir for a medicine, for example insulin. A supply means, such as a pump, is also provided in order to supply predetermined doses of the medicine through a conduit into the patient's body (not shown). The supply usually comprises a piston which is accommodated in the reservoir, wherein the reservoir can be replenished. Furthermore, a motor is coupled via a gear to the piston by a piston rod, in order to move the piston in the direction of the centre axis of the reservoir, which usually has a cylindrical shape, to push the medicine out of the reservoir. A power source is usually also included, for supplying power to the motor and other parts of the infusion device 10, such as a controller 14, a display 16, a button 18, a non-contact sensor or touch sensor 20 and a printed circuit board 24. The controller 14 also of course includes a processor, a memory portion including a part where non-erasable control data are stored, for instance a ROM memory, and a RAM and/or a reprogrammable memory such as an EEPROM, a flash memory or the like. The controller 14 can be accessed via the button 18, and data or information inputted via the button or requested from the controller can be indicated on the display 16.

A vibrator, a buzzer or loudspeaker or a combination of these can be used to provide a recognisable signal to the operator or patient. Instead of a loudspeaker or buzzer 26, it is of course also possible to use a visual signal which can be emitted by an LED or other device which provides a signal which can be recognised by a person such as a user or patient. The housing 22 of the infusion device 10 accommodates all the different components of the infusion device 10. It can be formed from different materials, for instance plastics or metal, but in the vicinity of the button 18 it is formed from a soft material which does not shield or deflect electrical, magnetic or electromagnetic fields.

In accordance with the invention, the non-contact sensor or touch sensor is either connected directly to the push button or button 18 in order to enable or disable the push button 18, or is connected to the controller 14 in order to enable or disable the button 18 via the controller 14. Furthermore, the sensor 20 can also output a signal in order to activate or deactivate the display 16.

When the sensor 20 is activated by an object such as the earthed finger of an operator or patient, the controller 14 can output a recognisable signal via the loudspeaker, buzzer, LED 26 or the like. This signal can be used to inform the operator or patient that the button 18 and/or the display 16 has been activated in order to input data, request data, or display information, respectively.

Figures 2a and 2b show a second embodiment in accordance with the present invention. The top view in Figure 2a shows the housing 22, which is made from a hard plastic material, metal or other suitable material or materials which are resistant to mechanical shocks. A soft material portion 18a is provided, which allows an operator to push the button 18 shown in Figure 2b, and which also enables an electrical field to extend through the soft material portion 18a, such that a person's finger can interact with the electrical field generated by the sensor 20 which, in the present case, can be a capacitive sensor. Accordingly, if an earthed portion of a person's body, for example a finger, approaches the sensor 20 of the infusion device 10', and the interaction is strong enough, the sensor 20 outputs an activation signal which enables the button 18. Both the button 18 and the sensor 20 can be mounted on a printed circuit board which can also support the electrical circuits of the controller 14.

Instead of a push button 18, it is also possible to use only a non-contact sensor or touch sensor in order to activate an input state and to input control data or request the display of data. This could be achieved using a particular code which is inputted by the operator or patient. For instance, the patient approaches the soft material portion two or three times, which is a code for activation, and if the code is accepted, the controller outputs a recognisable signal via the loudspeaker, buzzer or LED 26 shown in Figure 1, in order to inform the patient that the input state has been activated.

It is also for example possible to activate different input states or functions using different codes.

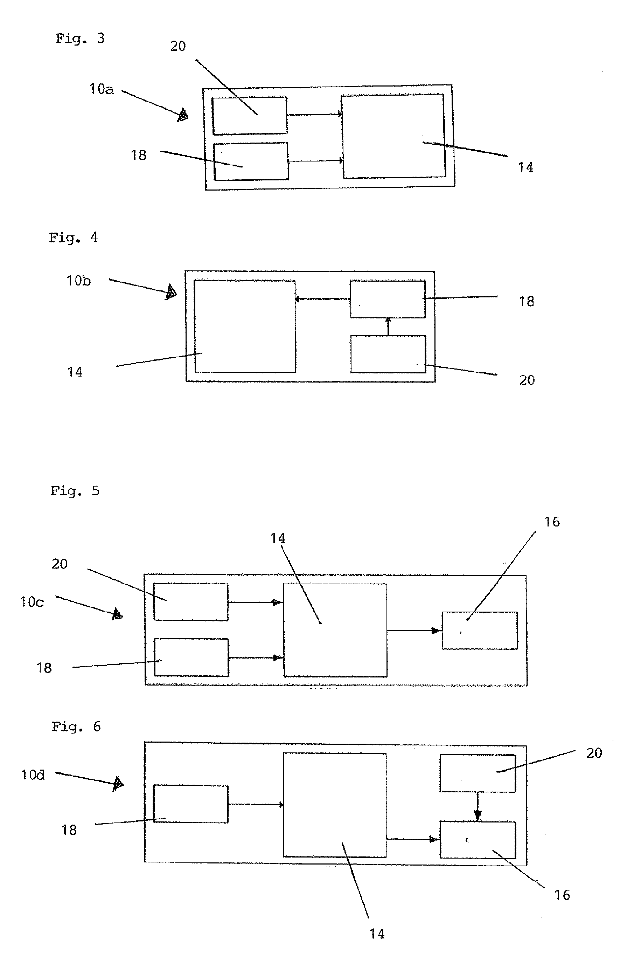

Figure 3 shows only a part of the infusion device 10a, namely the controller 14, the non-contact sensor or touch sensor 20 and the button or push button 18. In the embodiment of Figure 3, if the sensor 20 outputs an activation signal, the controller 14 then activates the button 18 or accepts signals inputted via the button 18. Again, it is possible for the controller 14 to allow signals to be inputted from the button 18 only for a predetermined but adjustable period of time of for instance several seconds, in order to prevent an unintended activation of the sensor 20 leading to signals being accepted from the button 18 over a long period of time, which could cause a bolus dose to be unintentionally administered.

In accordance with the embodiment of the device 10b in accordance with Figure 4, the sensor 20 directly activates the button or switch 18 and allows an operator or patient to input data or requests into the controller 14 via the button 18.

In accordance with the embodiment of the device 10c illustrated in Figure 5, the sensor 20 can output an activation signal which is received by the controller 14. Upon receiving the activation signal from the sensor 20, the controller 14 activates a display 16. As also shown in Figure 3, the controller 14 can simultaneously also activate the button 18, although in accordance with the embodiment in Figure 5, this is merely an option, and it is also possible for the display 16 to be activated only by the activation signal 3 outputted by the non-contact sensor or touch sensor 20.

The embodiment in Figure 6 relates to an infusion device 10d in which the non-contact sensor or touch sensor 20 directly activates the display 16, which means that data or information to be displayed are provided to the display, but only when the sensor 20 initiates the supply of electrical energy to the display 16. One or more buttons 18 can be used separately, without influencing the activation signal outputted by the sensor 20. On the other hand, it is of course also possible for the activation signal from the sensor 20 to also activate the button or buttons 18 for inputting information or data into the controller 14, wherein the activation signal enables the buttons to input information or requests.

## Claims

1. An administering device (10) for supplying an injectable or infusible product into an organism, in particular a patient, comprising:
- a reservoir for storing the product; and
- a supply device for supplying the product from the reservoir into the organism;
**characterised by**:
- a non-contact sensor or touch sensor for generating an activation signal for initiating a function of the device.

2. The device according to claim 1, wherein the device includes at least one button (18) for causing the device to be instructed to conduct an action, wherein the button (18) is enabled by the activation signal.

3. The device according to any one of claims 1 or 2, wherein the device includes a display (16) which is activated by the activation signal.

4. The device according to any one of claims 1 to 3, wherein the non-contact sensor or touch sensor is a capacitive or inductive sensor.

5. The device according to claims 1 to 3, wherein the device comprises a housing (22) which is formed, at least in the vicinity (18a) of the sensor (20), from a material which does not absorb or shield electrical, electromagnetic or magnetic fields.

6. The device according to any one of claims 1 to 3 or 5, wherein the non-contact sensor or touch sensor is a sensor which detects a change in resistance, in a conduction value or in light intensity.

7. The device according to any one of the aforementioned claims, wherein a control device (14) is provided which enables and/or disables functions of the device within a predetermined period of time after receiving the activation signal.

8. The device according to any one of claims 1 to 7, wherein the non-contact sensor or touch sensor (20) is configured to be activated and/or deactivated and/or to be variable with respect to its sensitivity.

9. The device according to any one of claims 1 to 8, wherein the non-contact sensor or touch sensor (20) is incorporated into the button (18) or is located in the vicinity of the button (18, 18a).

10. The device according to any one of claims 1 to 9, wherein the activation and/or deactivation of the non-contact sensor or touch sensor (20) and/or of the button (18) and/or of the display (16) is communicated to a user or operator of the device via an alert device (16).

11. The device according to any one of claims 1 to 10, wherein a valid activation of the button is communicated to a user or operator of the device.

12. The device according to any one of claims 1 or 3 to 8 or 10, wherein the controller is configured to accept a code from the sensor (20) and input data inputted via the sensor (20), once it has received the code.

13. The device according to one of claims 2 to 12, wherein the at least one button (18) includes the incorporated non-contact sensor or touch sensor such that a first function of the device is operated therewith while a second function of the device is activatable through the button (18).
